Europäisches Patentamt

European Patent Office

Office européen des brevets

(19)

(11) Veröffentlichungsnummer : **0 075 776**
**B1**

(12) **EUROPÄISCHE PATENTSCHRIFT**

(45) Veröffentlichungstag der Patentschrift :
07.11.84

(21) Anmeldenummer : **82108438.1**

(22) Anmeldetag : **13.09.82**

(51) Int. Cl.³ : **C 07 C103/52, A 61 K 37/02**

(54) **Bis-thymosin Alpha 1-Verbindungen.**

(30) Priorität : **18.09.81 DE 3137231**

(43) Veröffentlichungstag der Anmeldung :
**06.04.83 Patentblatt 83/14**

(45) Bekanntmachung des Hinweises auf die Patenterteilung : **07.11.84 Patentblatt 84/45**

(84) Benannte Vertragsstaaten :
**AT BE CH DE FR GB IT LI LU NL SE**

(56) Entgegenhaltungen :
**EP-A- 0 033 384**
**DE-A- 2 748 213**

(73) Patentinhaber : **Birr, Christian, Dr., Prof.**
**Werderstrasse 35**
**D-6900 Heidelberg (DE)**

(72) Erfinder : **Birr, Christian, Dr., Prof.**
**Werderstrasse 35**
**D-6900 Heidelberg (DE)**

(74) Vertreter : **Weickmann, Heinrich, Dipl.-Ing. et al
Patentanwälte Dipl.-Ing. H. Weickmann Dipl.-Phys.
Dr. K. Flincke Dipl.-Ing. F.A. Weickmann Dipl.-
Chem. B. Huber Dr.-Ing. H. Liska Dipl.-Phys. Dr. J.
Prechtel
Postfach 860820
D-8000 München 86 (DE)**

Jouve, 18, rue St-Denis, 75001 Paris, France

## Beschreibung

Die Erfindung betrifft Bis-thymosin $\alpha_1$-Verbindungen, Verfahren zu ihrer Herstellung und ihre Verwendung.

Thymosin $\alpha_1$ ist ein aus Thymus isoliertes Peptid, welches auch schon synthetisch hergestellt wurde (DE-OS 29 19 592).

Thymosin $\alpha_1$ stimuliert die Differenzierung und Vermehrung von T-Lymphozyten. Die Aktivierung der Immunabwehrzellen erfolgt durch Addition an Rezeptoren an der Zelloberfläche. Aufgrund dieser Eigenschaften hat Thymosin große Bedeutung als Stimulator der Immunabwehr, insbesondere auch bei der Krebsbekämpfung gefunden.

Nunmehr wurde gefunden, daß sich die Wirksamkeit von Thymosin $\alpha_1$ stark erhöhen läßt, wenn es in Form eines Zwillingsmoleküls vorliegt, in dem die Struktur des monomeren Thymosin $\alpha_1$ praktisch unverändert enthalten ist.

Gegenstand der Erfindung ist daher Bis-thymosin $\alpha_1$, welches dadurch gekennzeichnet ist, daß es aus zwei Desacetyl-thymosin $\alpha_1$-Einheiten besteht, die N-terminal durch eine Brückenverbindung mit 1 bis 10 Kohlenstoffatomen verknüpft sind.

Es wird angenommen, daß die überlegene Wirksamkeit des Bis-thymosin $\alpha_1$ darauf beruht, daß die Konzentration der Thymosin $\alpha_1$-Einheit am Wirkort stark erhöht wird, wenn man das erfindungsgemäße Derivat einsetzt.

Bevorzugt werden erfindungsgemäß Verbindungen der allgemeinen Formel

$$T_2R,$$

in welcher T einen Desacetyl-thymosin $\alpha_1$-Rest und R einen $\alpha,\omega$-Diacylrest mit 2 bis 10 C-Atomen, der gegebenenfalls einen aromatischen Kern, eine oder mehrere OH-Gruppen, $NH_2$-Gruppen, Halogenatome oder/und olefinische Doppelbindungen enthalten kann, oder eine CO-Gruppe bedeuten.

Der die beiden Thymosin $\alpha_1$-Reste verbindende Rest R leitet sich vorzugsweise von Phosgen, Dicarbonsäuren, wie Oxalsäure, Malonsäure, Bernsteinsäure, Glutarsäure, Adipinsäure, Asparaginsäure, Glutaminsäure, Maleinsäure, Fumarsäure, Äpfelsäure, Weinsäure, Terephthalsäure, Phenylendiessigsäure und ähnlichen ab. Bevorzugt leitet sich R von Phosgen, Oxalsäure, Bernsteinsäure, Asparaginsäure, Glutaminsäure, Maleinsäure oder Phenylendiessigsäure ab. Besonders bevorzugt wird Succinyl-bis-desacetylthymosin $\alpha_1$. Diese Verbindung leitet sich vom Thymosin $\alpha_1$, welches in seiner natürlichen Form $N^\alpha$-acetyliert vorliegt, ab durch Ersatz eines Wasserstoffatoms an der $CH_3$-Gruppe des Acetylrests durch eine Bindung mit dem zweiten Thymosin $\alpha_1$-Molekül.

Die Herstellung der neuen Bis-thymosin $\alpha_1$-Verbindungen erfolgt erfindungsgemäß dadurch, daß man ein synthetisches Desacetylthymosin $\alpha_1$, in dem alle funktionellen Gruppen der Aminosäuren 1 bis 28 durch in der Peptidsynthesetechnik übliche Schutzgruppen blockiert sind, ausgenommen die N-terminale Aminogruppe, mit einer difunktionellen, mit $NH_2$-Gruppen reaktiven Kupplungsverbindungen mit 1 bis 10 Kohlenstoffatomen in einem polaren Lösungsmittel umsetzt und anschließend die Schutzgruppen in an sich bekannter Weise abspaltet.

Zweckmäßig geht man hierbei von einer Vorstufe von synthetisch hergestelltem Thymosin $\alpha_1$ aus, bei welcher alle funktionellen Gruppen noch blockiert sind, jedoch die N-terminale Acetylgruppe noch fehlt. Dieses N-terminal ungeschützte Thymosin $\alpha_1$-Derivat wird zweckmäßig mit 0,5 Äquivalenten der difunktionellen Kupplungsverbindung umgesetzt, so daß die Bis-Amidierung sichergestellt ist. Überschüssiges blockiertes Desacetyl-thymosin $\alpha_1$ läßt sich leicht durch Acetylierung, z. B. mittels Acetylchlorid, in normales Thymosin $\alpha_1$ überführen, welches sich vom Dimeren säulenchromatographisch, z. B. in Trifluoräthanol, aufgrund des großen Molekulargewichtsunterschieds leicht abtrennen und schließlich durch Schutzgruppenabspaltung in die biologisch aktive Form überführen läßt.

Als synthetisches Desacetyl-thymosin $\alpha_1$-Derivat mit blockierten funktionellen Gruppen wird vorzugsweise Ddz(1-28)-OBzl-Thymosin $\alpha_1$ oder Ser(Bzl)-Asp(OBzl)-Ala-Ala-Val-Asp(OBzl)-Thr(Bzl)-Ser(Bzl)-Ser(Bzl)-Glu(OBzl)-Ile-Thr(Bzl)-Thr-(Bzl)-Lys(Z)-Asp(OBzl)-Leu-Lys(Z)-Glu(OBzl)-Lys(Z)-Lys(Z)-Glu(OBzl)-Val-Val-Glu(OBzl)-Glu(OBzl)-Ala-Glu(OBzl)-Asn-OBzl verwendet. Diese Verbindungen sind bekannt aus DE-OS 29 19 592 bzw. JACS *101*, 253-254 (1979).

Wird das Ddz-schutzgruppenblockierte Derivat verwendet, so erfolgt die Schutzgruppenabspaltung durch Behandlung mit einer Säure in einem polaren organischen Lösungsmittel. Als Säure wird eine Trialkylessigsäure wie Trichloressigsäure oder Trifluoressigsäure bevorzugt.

Als Kupplungsverbindung eignen sich difunktionelle reaktionsfähige Verbindungen mit 1 bis 10 Kohlenstoffatomen. Bevorzugt werden reaktive Dicarbonsäure-Derivate wie Diaktivester Halogenide, insbesondere Chloride, und Azide, Phosgen und Diepoxide verwendet. Typische Beispiele für derartige reaktive Dicarbonsäureverbindungen sind Oxalsäuredichlorid, Malonsäure-dichlorid, Bernsteinsäuredichlorid oder -anhydrid, Glutarsäure-dichlorid, Adipinsäure-dichlorid, Asparaginsäure-dichlorid, Glutaminsäure-dichlorid, Maleinsäure- bzw. Fumarsäure-dichlorid, Äpfelsäure-dichlorid, Weinsäure-dichlorid, Phthalsäure-dichloride, Phenylendiessigsäure-dichlorid sowie die entsprechenden Verbindungen, die sich von der Chlorbernsteinsäure, Thymidinsäure, Korksäure, Azelainsäure, Sebacinsäure, Oxyglytaminsäure, Itakonsäure und Traubensäure ableiten. Die Dicarbonsäuren können auch in Form ihrer Aktivester, z. B. als p-Nitrophenyl-, Trichlorphenyl-, Pentachlorphenyl- oder Pentafluorphenylester eingesetzt werden. Außerdem ist es möglich, auch die freien Dicarbonsäuren direkt zusammen mit

a) einem Carbodiimid, wie Cyclohexylcarbodiimid, oder Carbonylbisimidazol und
b) Benzotriazol einzusetzen.

Ferner können von den Dicarbonsäuren abgeleitete Dialdehyde verwendet werden. Die hierbei gebildete Schiff'sche Base wird dann zum sekundären Amin hydriert.

Eine bevorzugte Kupplungsverbindung ist Glutardialdehyd.

Für die Reaktion zwischen der difunktionellen Brückenbildnerverbindung und der Aminogruppe des Desacetyl-thymosin $\alpha_1$ werden die hierfür bekannten Reaktionsbedingungen angewendet. Beispiele für geeignete Lösungsmittel sind Dimethylsulfoxid, Dimethylformamid, Formamid und ähnliche sowie deren Gemische. Zweckmäßig erfolgt die Umsetzung in Gegenwart einer äquivalenten Menge einer Base wie N-Methylmorpholin.

Die Herstellung der bevorzugten Ausgangsmaterialien ist beschrieben in Angew. Chemie *91*, 422-423 (1979) bzw. JACS *101*, 253-254 (1979).

Das folgende Beispiel erläutert die Erfindung weiter.


### Beispiel

50 mg vollgeschützte, synthetische Vorstufe von Thymosin $\alpha_1$, Ddz-(1-28) OBzl (10 µMol ; MG : 5040), hergestellt wie in Angew. Chemie *91*, 422-423 (1979) beschrieben, werden in 5 ml Dichlormethan unter magnetischem Rühren mit 37 µl (475 µMol) Trifluoressigsäure versetzt und für 1 h bei 20° bei Feuchteausschluß belassen. Dünnschichtchromatographische Kontrolle in Trichlormethan/Methanol 9 : 1 zeigt Ddz-Schutzgruppenabspaltung anhand des wandernden Ddz-Spaltstückes. Der Ansatz wird mit 55 µl (480 µMol) N-Methylmorpholin neutralisiert und im Vakuum bei 40° eingedampft. Vom unlöslichen wird das Ddz-Spaltstück durch Digerieren mit Benzin (40°-Frakt.) abgewaschen und der Rückstand im Vakuum bei 40° kurz getrocknet. Dann wird mit 5 ml Wasser versetzt und das in Schuppen aufschwimmende, N-terminal freie $H_2N$-(1-28) OBzl durch Zentrifugation gewonnen. Das so abgetrennte Peptid wird über $P_2O_5$/KOH im Vakuum getrocknet und zur Weiterverwendung aufbewahrt.

10 µMol $H_2N$-(1-28) OBzl werden in 1 ml Dimethylsulfoxid bei 40° gelöst und auf 0° abgekühlt und mit 1.11 µl (10 µMol) N-Methylmorpholin versetzt. Hierein wird bei 0° im Ultraschallbad die Lösung von 0.283 µl (2.5 µMol ; 0.5 Äquiv.) Succinoyldichlorid in 100 µl Dimethylformamid (0°) eingetropft und 1 h bei 0° sowie 5 h bei 40° im Ultraschallbad umgesetzt. Anschließend wird auf 0° abgekühlt, mit 2.22 µl N-Methylmorpholin versetzt und mit 1.43 µl (20 µMol) Acetylchlorid bei 0° im Ultraschallbad acetyliert, 1 h 0°, 5 h 20-30°.

Zur Aufarbeitung wird der Ansatz unter magnetischem Rühren in 150 ml Wasser von 0° eingetragen und dabei das Produkt gefällt. Die Fällung wird nach 2 h auf einer G4-Fritte abgesaugt und mit Eiswasser gewaschen. Der damit salz- und säurechloridfreie Filterrückstand wird über $P_2O_5$ getrocknet und über eine Sephadex® LH20/Trifluorethanol-Säule (1 × 200 cm) chromatographiert. Die Abspaltung der Schutzgruppen erfolgt durch Hydrogenolyse in 2,2,2-Trifluorethanol mit Pd/C (Benzyloxycarbonyl- und C-terminale Benzylestergruppen), dann 30 Minuten Einwirkung von Trifluoressigsäure/Dichlormethan 1 : 1 (V/V), enthaltend 10 Vol.-% Anisol (t-Butylestergruppen), Einengen im Vakuum und schließlich Einwirkung reiner Trifluoressigsäure während ca. 2 Stunden bei Raumtemperatur (4,4'-Dimethoxybenzhydryl- und t-Butylgruppen). Die chromatographische Identifizierung und Isolierung des freien, dimeren $N^\alpha$-Succinoyl-bis-thymosin $\alpha_1$ (MG 6212) erfolgt ebenfalls wie dort beschrieben an einer für das Molekulargewicht von oxidierter Insulin B-Kette (MG 3495) geeichten und mit Thymosin $\alpha_1$ (MG 3107) getesteten Biogel P6-Säule (0.6 × 240 cm) in Wasser (10 % Trifluorethanol, 1 % Essigsäure).

Dünnschichtchromatographisch wandert $N^\alpha$-Succinoyl-bis-thymosin $\alpha_1$ nicht ($R_f 0$), während im Vergleich hierzu das monomere Thymosin $\alpha_1$ einen $R_f = 0.16$ zeigt (Laufmittel : n-Butanol/Pyridin/Eisessig/Wasser 5 : 5 : 1 : 4 ; V/V).

Die nachfolgende Tabelle 1 zeigt die Stimulation (in %) peripherer Human-T-Lymphocyten in der « Gemischten Lymphocyten Kultur » (MLC) durch ein erfindungsgemäßes Bis-Thymosin-$\alpha_1$ ($N^\alpha$-Succinoyl-bis-thymosin-$\alpha_1$) und durch Thymosin-$\alpha_1$.


### Tabelle 1

| Wirkstoff (0,5 µg/Kultur) | Bis-Thymosin-$\alpha_1$ | Thymosin-$\alpha_1$ |
|---|---|---|
| Stimulation in % (ohne Wirkstoff = 100) | 162 | 126-141 |

Die Tabelle zeigt, daß durch einen Zusatz von 0,5 µg Bis-Thymosin-$\alpha_1$ im Vergleich zur Normalstimulation ohne Wirkstoff (= 100 %) sowie zur Stimulation durch die Ausgangssubstanz Thymosin-$\alpha_1$ (126-141 %) eine hohe Überschußstimulation (162 %) bewirkt wird.

In der Tabelle 2 ist die Stimulation peripherer Human-T-Lymphozyten im E-Rosettentest mit Vorinhibition durch 0,2 µg α-Amanitin/Kultur nach Zusatz der zu testenden Wirkstoffe angegeben (Normal-E-Rosettenzahl ohne Zusatz = 100 % ; E-Rosettenzahl nach Vorinhibition mit α-Amanitin = 0 %). Gemessen wird die Fähigkeit der Wirkstoffe, in der α-Amanitin-inhibierten Kultur die Normal-E-Rosettenzahl durch Stimulation der T-Lymphocyten wieder zu restaurieren.

Tabelle 2

| Wirkstoff (5 µg/Kultur) | Bis-Thymosin-$\alpha_1$ | Thymosin-$\alpha_1$ |
| --- | --- | --- |
| Stimulation (in %) | 95 | 80-90 |

Die Tabelle zeigt, daß trotz des doppelten Molekulargewichts von Bis-Thymosin-$\alpha_1$ die größere Wirkdichte von erfindungsgemäßem Bis-thymosin-$\alpha_1$ am Rezeptor bereits signifikant zum Ausdruck kommt, wenn alle getesteten Wirkstoffe den Zellen in gleicher Menge zugegeben werden.

Die Tests der Tabellen 1 und 2 wurden nach C. Birr et. al, PEPTIDES, Synthesis-Structure-Function, Proc. 7th. Amer. Peptide Symp. (D. H. Rich and E. Gross, Eds.) Pierce Chem. Comp., Rockford, U.S.A., 1981, pp. 545-548 durchgeführt.

**Ansprüche**

1. Bis-thymosin $\alpha_1$, dadurch gekennzeichnet, daß es aus zwei Desacetyl-thymosin $\alpha_1$-Einheiten besteht, die N-terminal durch eine Brückenverbindung mit 1 bis 10 Kohlenstoffatomen verknüpft sind.

2. Verbindung nach Anspruch 1, gekennzeichnet durch die allgemeine Formel

$$T_2R,$$

in welcher T einen Desacetyl-thymosin $\alpha_1$-Rest und R einen $\alpha,\omega$-Diacylrest mit 2 bis 10 C-Atomen, der gegebenenfalls einen aromatischen Kern, eine oder mehrere OH-Gruppen, $NH_2$-Gruppen, Halogenatome oder/und olefinische Doppelbindungen enthalten kann, oder eine CO-Gruppe bedeuten.

3. Verbindung nach Anspruch 2, dadurch gekennzeichnet, daß R sich von Phosgen, Oxalsäure, Bernsteinsäure, Asparaginsäure, Glutaminsäure, Maleinsäure oder Phenylendiessigsäure ableitet.

4. Succinyl-bisdesacetyl-thymosin $\alpha_1$.

5. Verfahren zur Herstellung einer Verbindung gemäß Anspruch 1 bis 4, dadurch gekennzeichnet, daß man ein synthetisches Desacetyl-thymosin $\alpha_1$, in dem alle funktionellen Gruppen der Aminosäuren 1 bis 28 durch in der Peptidsynthesetechnik übliche Schutzgruppen blockiert sind, ausgenommen die N-terminale Aminogruppe, mit einer difunktionellen, mit $NH_2$-Gruppen reaktiven Kupplungsverbindungen mit 1 bis 10 Kohlenstoffatomen in einem polaren Lösungsmittel umsetzt und anschließend die Schutzgruppen in an sich bekannter Weise abspaltet.

6. Verfahren nach Anspruch 5, dadurch gekennzeichnet, daß man als synthetisches Desacetyl-thymosin $\alpha_1$-Derivat mit blockierten funktionellen Gruppen Ddz(1-28) OBzl-thymosin $\alpha_1$ oder Ser(Bzl)-Asp(OBzl)-Ala-Ala-Val-Asp(OBzl)-Thr(Bzl)-Ser(Bzl)-Ser(Bzl)-Glu(OBzl)-Ile-Thr(Bzl)-Thr-(Bzl)-Lys(Z)-Asp(OBzl)-Leu-Lys(Z)-Glu(OBzl)-Lys(Z)-Lys(Z)-Glu(OBzl)-Val-Val-Glu(OBzl)-Glu(OBzl)-Ala-Glu(OBzl)-Asn-OBzl verwendet.

7. Verfahren nach Anspruch 5 oder 6, dadurch gekennzeichnet, daß man als Kupplungsverbindung Phosgen, ein aktiviertes Dicarbonsäurederivat, wie ein Halogenid oder Azid, oder ein Diepoxid verwendet.

8. Verfahren nach Anspruch 5 oder 6, dadurch gekennzeichnet, daß man als Kupplungsverbindung einen Dialdehyd verwendet und die gebildete Schiff'sche Base unter Bildung des sekundären Amins hydriert.

9. Mittel zur Stimulierung der Immunabwehrzellen, dadurch gekennzeichnet, daß es aus einer Verbindung nach einem der Ansprüche 1 bis 4 als Wirkstoff zusammen mit üblichen pharmazeutischen Konfektionierungsmitteln, Verdünnungsmitteln oder/und Adjuvantien besteht.

**Claims**

1. Bis-thymosin $\alpha_1$, characterised in that is consists of two desacetyl-thymosin $\alpha_1$ units, which are N-terminally linked by a bridge compound with 1 to 10 carbon atoms.

2. Compound according to claim 1, characterised by the general formula

$$T_2R,$$

in which T signifies a desacetyl-thymosin $\alpha_1$ residue and R an $\alpha, \omega$-diaceyl radical with 2 to 10 C-atoms which can optionally contain an aromatic nucleus, one or more OH groups, NH$_2$ groups, halogen atoms and/or olefinic double bonds, or a CO group.

3. Compound according to claim 2, characterised in that R is derived from phosgene, oxalic acid, succinic acid, aspartic acid, glutamic acid, maleic acid or phenylene-diacetic acid.

4. Succinyl-bis-desacetyl-thymosin $\alpha_1$.

5. Process for the preparation of a compound according to claims 1 to 4, characterised in that one reacts a synthetic desacetyl-thymosin $\alpha_1$, in which all functional groups of the amino acids 1 to 28 are blocked by protective groups conventional in peptide synthesis chemistry, excepting the N-terminal amino group, with a difunctional coupling compound with 1 to 10 carbon atoms reactive with NH$_2$ groups and subsequently splits off the protective groups in known manner.

6. Process according to claim 5, characterised in that, as synthetic desacetyl-thymosin $\alpha_1$ derivative with blocked functional groups, one uses Ddz(1-28)-OBzl-thymosin $\alpha_1$ or Ser(Bzl)-Asp(OBzl)-Ala-Ala-Val-Asp(OBzl)-Thr(OBzl)-Ser(Bzl)-Ser(Bzl)-Glu(OBzl)-Ile-Thr(Bzl)-Thr-(Bzl)-Lys(Z)-Asp(OBzl)-Leu-Lys(Z)-Glu(OBzl)-Lys(Z)-Glu(OBzl)-Val-Val-Glu(OBzl)-Glu(OBzl)-Ala-Glu(OBzl)-Asn-OBzl.

7. Process according to claim 5 or 6, characterised in that as coupling compound one uses phosgene, an activated dicarboxylic acid derivative, such as a halide or azide, or a diepoxide.

8. Process according to claim 5 or 6, characterised in that as coupling compound one uses a dialdehyde and hydrogenates the Schiff's base formed with the formation of the secondary amine.

9. Agent for the stimulation of immune defence cells, characterised in that it consists of a compound according to one of claims 1 to 4 as active material, together with conventional pharmaceutical confectioning agents, dilution agents and/or adjuvants.


## Revendications

1. Bis-thymosine alpha 1, caractérisée en ce qu'elle consiste en deux motifs de désacétyl-thymosine alpha 1 reliés sur l'azote terminal par un composé en C1-C10 formant un pont.

2. Composé selon la revendication 1, caractérisé par la formule générale

$$T_2R,$$

dans laquelle T représente un reste de désacétyl-thymosine alpha 1 et R un reste alpha, oméga-diacyle en C2-C10 qui peut contenir le cas échéant un noyau aromatique, un ou plusieurs groupes OH, NH$_2$, atomes d'halogènes et/ou doubles liaisons oléfiniques, ou un groupe CO.

3. Composé selon la revendication 2, caractérisé en ce que R dérive du phosgène, de l'acide oxalique, de l'acide succinique, de l'acide aspartique, de l'acide glutamique, de l'acide maléique, ou de l'acide phénylène-diacétique.

4. La succinyl-bis-désacétyl-thymosine alpha 1.

5. Procédé de préparation d'un composé selon les revendications 1 à 4, caractérisé en ce que l'on fait réagir une désacétyl-thymosine alpha 1 synthétique, dans laquelle tous les groupes fonctionnels des amino-acides 1 à 28 sont bloqués par des groupes protecteurs usuels dans les techniques de synthèse des peptides, à l'exception du groupe amino à N terminal, avec un agent couplant difonctionnel en C1-C10 réactif avec les groupes NH$_2$ dans un solvant polaire après quoi on élimine les groupes protecteurs de manière connue en soi.

6. Procédé selon la revendication 5, caractérisé en ce qu'on utilise en tant que dérivé synthétique de désacétyl-thymosine alpha 1 à groupes fonctionnels bloqués la Ddz(1-28)-OBzl-thymosine alpha 1 ou Ser(Bzl)-Asp(OBzl)-Ala-Ala-Val-Asp(OBzl)-Thr(Bzl)-Ser(Bzl)-Ser(Bzl)-Glu(OBzl)-Ile-Thr(Bzl)-Thr-(Bzl)-Lys-(Z)-Asp(OBzl)-Leu-Lys(Z)-Glu(OBzl)-Lys(Z)-Lys(Z)-Glu(OBzl)-Val-Val-Glu(OBzl)-Glu(OBzl)-Ala-Glu-(OBzl)-Asn-OBzl.

7. Procédé selon la revendication 5 ou 6, caractérisé en ce que l'on utilise en tant qu'agent couplant le phosgène, un dérivé activé d'acide dicarboxylique tel qu'un halogénure ou un azide, ou un diépoxyde.

8. Procédé selon la revendication 5 ou 6, caractérisé en ce que l'on utilise en tant qu'agent couplant un dialdéhyde et on hydrogène la base de Schiff obtenue en formant l'amine secondaire.

9. Agent pour la stimulation des cellules de défense immunitaire, caractérisé en ce qu'il consiste en un composé selon l'une des revendications 1 à 4 en tant que substance active, avec des agents de confectionnement, des diluants et/ou des adjuvants pharmaceutiques usuels.